# EUROPEAN PATENT APPLICATION

(11) **EP 2 002 840 A2**
(43) Date of publication of application: **17.12.2008**
(21) Application number: 06769535.3
(22) Date of filing: 27.03.2006
(51) Int. Cl.: A61K 31/711, A61P 35/00

(54) **METHOD FOR TREATING ONCOLOGICAL DISEASES**

(30) Priority: 16.01.2006 RU 2006100561
(71) Applicant: Shurdov, Mikhail Arkadievich, Moscow 124305 (RU)
(72) Inventor: BOGACHEV, Sergey Stanislavovich, Novosibirsk, 630058 (RU); YAKUBOV, Leonid Anatolievich, Novosibirsk, 630090 (RU); ROGACHEV, Vladimir Alexeevich, Novosibirsk, 630106 (RU); NIKOLIN, Valery Petrovich, Novosibirsk, 630090 (RU); ZHDANOVA, Natalia Sergeevna, Novosibirsk, 630090 (RU); POPOVA, Nelly Alexandrovna, Novosibirsk, 630090 (RU); LIKHACHEVA, Anastasia Sergeevna, Novosibirsk, 630058 (RU); SEBELEVA, Tamara Egorovna, Novosibirsk, 630090 (RU); SHILOV, Alexander Gennadievich, Novosibirsk, 630090 (RU); MECHETINA, Ljudmila Vasilievna, Novosibirsk, 630090 (RU); VRATSKIKH, Oxana Vyacheslavovna, Novosibirsk, 630090 (RU); SEREGIN, Sergey Nikolaevich, Moscow, 124460 (RU)
(74) Representative: Gerasimovic, Liudmila
(86) International application number: PCT/RU2006/000144
(87) International publication number: WO 2007/081235

(57) **Abstract**

The invention belongs to the field of medicine, in particular, to the methods for treating the patients with the preparations containing genetic material, and may be used for treating the patients with oncologic diseases caused by mutations in oncogenes, oncosuppressor genes, and a total gene allele homozygotization of the cell that undergoes a malignant transformation.

The stated problem consisting in expansion of the area of application and treatment of patients with oncologic diseases caused by both the mutations in oncogenes and oncosuppressor genes and a total gene homozygotization is solved by that the fragments of homologous DNA comprising the complete genome of a physiologically and genetically healthy donor are introduced into the patient's organism and the quantity of the DNA introduced is equal to or exceeds the amount of the own DNA in the blood plasma and tissue fluids, but does not exceed the maximal permissible dose, amounting to 30 µg/ml.

## Description

### Field of invention

The invention belongs to the field of medicine, in particular, to the methods for treating the patients with the preparations containing genetic material, and may be used for treating the patients with oncologic diseases caused by mutations in oncogenes, oncosuppressor genes, and a total gene homozygotization of the cell that undergoes a malignant transformation.

### Background of the invention

A treatment method is known that is based on repair of point mutations in cells [US, No. 5,795,972, 18.08.1998.]. This method displays a relatively low treatment efficiency and has a limited range of administration, because this method requires that the mutations are precisely determined before commencement of the treatment.

Since a reliable proof that a particular mutation in the cause of a cancer is yet lacking, development of the method mentioned above requires a meticulous and long-term research into detection of particular mutations that require correction, thereby necessitating the development of the methods that would be applicable on the background of already available knowledge about causes of cancer without emphasis on particular changes in the genome that induced malignization. The methods involving a local administration of DNA fragments for treatment of precancerous states in the patient's skin and stimulation of suntan are also known [US, No. 5,955,059, 21.09.1999, US, No. 5,470,577, 28.11.1995].

The applicability of these methods is limited, because neither particular DNA sequences nor DNA sources in the corresponding patents are specified. They propose using both the natural and synthetic DNA from "any appropriate source", for example, salmon DNA with a length of 200 bp down to mononucleotides and nucleosides, including dimers, which are the most efficient according to the tests of the authors. However, the effect and consequences of administration of the foreign DNA on the human organism are yet to be studied in detail, which predetermines a certain danger in applying these methods.

The method that is the closest in its essence to the method proposed is based on activation of DNA repair in the cells; according to this method, repair enzymes are delivered to skin cells [US, No. 5,302,389, 12.04.1994].

The shortcoming of the closest technical solution is a limited area of application, since it is intended for treatment of only skin oncologic diseases and does not imply restoration of the already existing mutations.

All these demonstrate that the need in the methods for correction of genetic mutations is still most topical. To a considerable degree, it refers to the treatment of individuals with oncologic diseases caused by mutations in both the oncogenes and oncosuppressor genes as well as by total gene homozygotization.

### Substance of the invention

The problem to be solved is expansion of the application area and treatment of the patients with the oncologic diseases caused by mutations in both the oncogenes and oncosuppressor genes as well as by total gene homozygotization.

In a wider aspect, the technical problem of this invention is the development of the method to affect the cancer cells and overall tumor, experimentally confirmed in the models of human adenocarcinoma cell culture and artificially induced mouse tumors, in such a manner that the gene homeostasis is changed when the cells undergo reverse genetic transformation that causes the loss of the main feature of the cancer cell-its unlimited proliferative activity.

The required result is reached by that the fragmented DNA is introduced into the patient's organism for treatment of oncologic diseases, wherein the fragmented DNA is the fragments of homologous DNA with biologically active size and comprising the complete genome of a physiologically and genetically healthy donor. The fragmented DNA is used in amount which is equal to or exceeds the amount of the own DNA of blood plasma and tissue fluids of the patient, but is not more than the maximally permissible quantity equaling 30 µg/ml.

The modern literature lacks the references to the proposed method for treatment of the patients with oncologic diseases using fragmented genetically healthy DNA, natural mechanism of its delivery and deposition in the interchromosome space, and homologous exchange between the fragments delivered and the corresponding chromosome regions carrying the mutations that caused the cancer transformation of the particular cell.

Consequently, the proposal meets the criteria of novelty and invention level.

The theoretical and experimental data confirming that the invention meets also the criterion of practical (industrial) applicability are given below.

### Brief description of the drawings

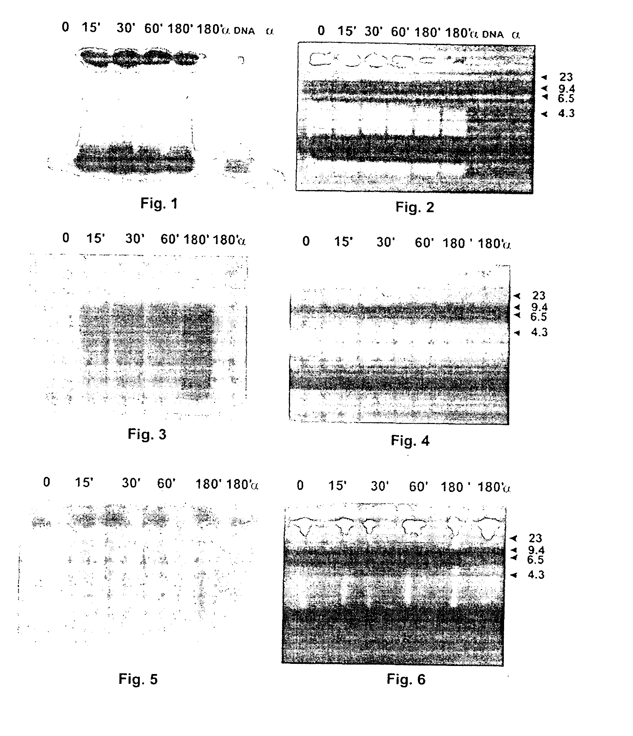

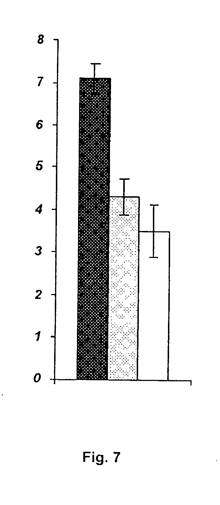

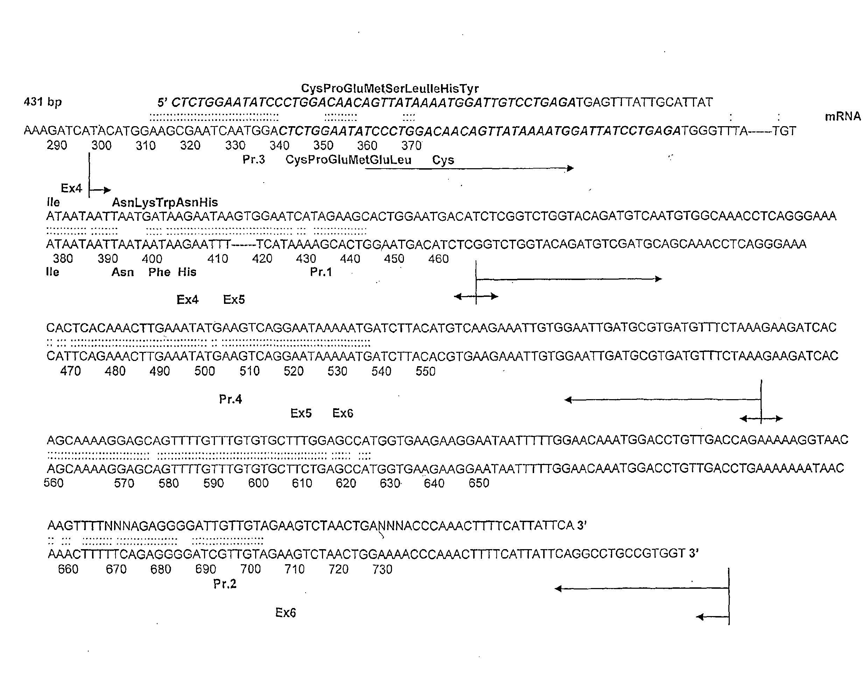

The fist page of the drawings shows the analysis of distribution of the fragmented human extracellular DNA between the cell compartments of MCF-7 cell culture depending on the time of cell exposure to the DNA in culture medium (native conditions of electrophoresis; logarithmic stage of culture growth). Fig. 1 and fig. 2 - Cytoplasmic fraction, fig. 3 and fig. 4 - , Interchromosomal fraction, fig. 5 and fig.6 -chromatin. Fig.1. Fig.2, Fig.3 - the agarose blocks stained with ethidium bromide are shown. Fig.2, Fig.4, Fig. 6 x-ray patterns on the same blocks after drying, leftward. Numbers above the blocks indicate the time of culture incubation with the labeled ethidium bromide and α³²p with extracellulr DNA and α-dATP*. The numbers to the right and left of the blocks, markers of molecular weight in kbp; α, the initial α-dATP* precursor. Fig. 7 shows the effect of the DNA preparation of the growth of Ehrlich ascites tumor.
- control tumor- black square-
- Fragmented DNA 400 ug x 1 -grey square-
- Fragmented DNA 100 ug x 4 -white square-

The healthy sequence and the sequence of specific region of caspase 3 mRNA that contained the repaired exon 4 and exons 5 and 6 also are represented. Indicated in the figure are the set of primers and the anticipated PCR fragment of caspase 3 gene with a size of 431 bp in RT procedure; the set of primers and the anticipated PCR fragments with sizes of 236 and 304 bp, confirming the attribution of the 431-bp fragment. Amplification of the 431-bp fragment demonstrates the existence of mRNA that is synthesized from the repaired caspase 3 gene. Amplification of the 236- and 304-bp fragments indicates that the internal structure of 431-bp fragment fits the corresponding region of caspase 3 mRNA, which contains the repaired exon 4 and exons 5 and 6. Comparison of the caspase 3 mRNA sequence, containing exons 4, 5, and 6, and the sequenced 236- and 304-bp fragments as internal segments obtained from the template of 431-bp fragment.

The proposed use of fragmented DNA for treatment of oncologic diseases is realized as follows.

Fragmented DNA is introduced into the patient's organism (by intravenous, intramuscular, or intracutaneous injections; subcutaneously; intraperitoneally; by washing or application to mucosae and/or skin; by oral administration with or without preliminary neutralization of the acid stomach medium or using a special capsule insoluble in the stomach; intrarectally; intravaginally; intraocularly; intrarhinally; by introduction into the tumor; intracerebrally; by intraocular injection; by inhalation, or injection into the cerebrospinal canal).

For this purpose, fragments of homologous DNA comprising the complete genome of physiologically and genetically healthy donor are used.

The quantity of the introduced fragmented DNA is equal or exceeds the amount of own DNA of the blood plasma and tissue fluids of the healthy donor; however, this amount is not more that the maximally permissible quantity, equaling 30 µg/ml.

In this process, the DNA fragments, whose size is biologically active, on entering the organism of the patient are delivered to the actively dividing cells of the body, including the cells that have undergone malignization, via natural delivery mechanisms (blood flow and specific receptors located on the surface of dividing cells and displaying a high affinity for the DNA fragments in question).

Then the DNA fragments that are delivered to the intracellular space are deposited in the interchromosomal compartment of the nucleus to undergo an intranuclear processing.

In this case, the DNA fragments deposited in the interchromosomal compartment of the nucleus substitute the mutant regions of the genes mutations in which have caused malignization of the cell with healthy regions according to the natural mechanism of homologous recombination as well as substitute the homozygotized alleles, a feature characteristic of the cells that underwent a malignant transformation, restoring their heterozygous condition, characteristic of the healthy state. As a result of these events, a reverse complete or partial transformation of the previously cancer cell takes place, thereby leading to the loss of the state of uncontrolled proliferation by this cell.

Thus, when homologous fragmented DNA from a physiologically and genetically healthy donor is introduced into the organism, it is taken up by actively dividing malignant cells via a natural delivery mechanism characteristic of actively dividing cells (receptor-mediated pinocytosis). The fragmented DNA delivered to the nuclear compartment-interchromosomal space-in deposited there and is involved in the process of homologous exchange with the corresponding chromosomal loci. Due to the natural mechanism of homologous recombination, the DNA fragments deposited with the interchromosomal space substitute the mutant regions of the genes, the mutations in which causes cell malignization, with the corresponding healthy fragments as well as the DNA fragments in question substitute the homozygotized alleles, a feature characteristic of the cells that underwent a malignant transformation, restoring their heterozygous condition, characteristic of the healthy state. As a result of these events, a reverse complete or partial transformation of the previously cancer cell takes place, thereby switching the cell to the development route of differentiated cell.

The effect that is reached when using the invention proposed may be justified by the following theoretical data.

Each cell of the organism contains genomic DNA, which codes for the information about all the proteins of all the organism's cells as well as about the spatial organization of the genes in the nucleus necessary for a correct spatiotemporal expression of all these proteins. Thus, genomic DNA is the template for the life of the organism, which contains the information about both the overall organism and its development. Initially, genomic DNA is identical in all the cells of the organism. However, with development of the organism, the genomic DNA in each cell mutates due to the effects of environmental factors and the errors occurring during cell replication and repair. It is known that the repair mechanisms substitute incorrect or impaired DNA bases and link again the ends of DNA molecule after single-strand or double-strand breaks (DSB) immediately after the mutation events. For this purpose, they may use the second DNA strand as a template. When the affected regions are very long and involve both DNA strands, the repair is problematical, and a mutation may emerge as a result of the impairment. Thus, mutagenic environmental factors and the mistakes of cell repair and replication are the source of somatic mutations in the cells. The modern scientific concept of carcinogenesis states that a gradual accumulation of mutations in cells causes their multistep cancer transformation.

The genetic multimutational nature of malignization of the cells and of the cancer itself suggests that the methods for treating cancer should be directed to elimination of the disease causes and first and foremost, to treatment (correction) of mutations. The only principle approach to treatment of the diseases connected with genetic impairments, to which oncologic diseases belong, is the change in the genome of malignant cells of the patient's organism by one or another method and restoration of the initial genetic homeostasis characteristic of the healthy cell. Numerous methods of gene therapy have been developed within the frames of this approach; however, all these methods are not free from serious shortcomings and ineffective in the context of a complex eukaryotic organism.

The classical methods of gene therapy (gene targeting) directed to correction of chromosomal mutations using viral constructs are capable of efficiently introducing the working copies of genes into the cell genome. Nonetheless, due to a random insertion of the gene and its regulatory regions, these approaches are not free from a number of problems related to the dependence of gene expression on the site of its integration, silencing of the introduced viral genome, and insertion mutagenesis associated with carcinogenesis [Khon, D.B., Sadelain M., Glorioso J.C. Occurrence of leukaemia following gene therapy of X-linked SCID. Nature Rev. Cancer. 477-488 (2003), Persons, D.A., Nienhuis, A.W. Gene therapy of hemoglobin disorders. Hematol. Rep. 2, 348-355 (2003)]. Correction or substitution of a mutant gene within the chromosome via its natural homologous recombination with the DNA template delivered to the cell that lacks the mutation in question could represent an attractive alternative to these methods, but for an extremely low efficiency of this approach demonstrated in early attempts of its application [Yanez, R.J. and Porter, A.C.G. Therapeutic gene targeting. Gene Therapy. 5, 149-159, (1998)., Rothstein, R.J. One-step gene disruption in yeast. Methods Enzymol. 101:202-211 (1983)].

Historically, the strategy of gene replacement developed with the aim to overcome the problems of insertion mutagenesis in yeasts and later, in mammalian cells, involved the use of linear recombinant DNA with its ends homologous to the regions flanking the target gene to be substitutes and the gene itself substituted with a selectable marker [Thomas, K.R., Capecchi, M.R. Site-directed mutagenesis by gene targeting in mouse embryo-derived stem cells. Cell. 51(3):503-512 (1987)]. This type of gene targeting is named "ends-out", because the ends of this construct correspond to the two sequences of the chromosomal DNA coming out of the target gene. In this process, the ends of the ends-out gene targeting construct are recombinogenic and facilitate the substitution of the gene sequence located between the terminal homologous regions. It is recently demonstrated that when substituting a complete gene with a heterologous sequence or substituting a single mutated base pair by this method in yeasts, the process is initiated by two independent strand invasions, which implies a search for homology and recombinational events in which the two terminal segments of the therapeutic construct are involved [Langston, L.D., Symingtom, L.S. Gene targeting in yeast is initiated by two independent strand invasions. Proc NM Acad Sci USA. 101(43): 15392-15397 (2004)]. Earlier, this mechanism was proposed for explanation of the ends-out gene targeting in mammalian cells [Li, J., Read, L.R., Baker, M.D. The mechanism of mammalian gene replacement is consistent with the formation of long regions of heteroduplex DNA associated with two crossing-over events. Mol. Cell Biol. 21(2):501-510 (2001)]. The somatic homologous recombination must evidently perform an absolutely necessary function universal for all the kingdoms of living organisms and perform it efficiently, since it has been evolutionarily preserved for billions of years. As the somatic homologous recombination in unicellular yeasts is the main mechanism of precision DSB reparation, its efficiency must be very high to provide the rescue of individual. Early experiments with microinjection of plasmids into the cell nucleus [Capecchi, M. Generating mice with targeted mutations. Nature Medicine 7, 1086-1090. (2001)] led to the discovery that the plasmids in all the cases integrated into the cell genome in a form of the single concatemer composed of all (frequently more than one hundred) injected plasmids linked into one molecule in a tail-to-head manner and that the result of this was n amazingly efficient work of the homologous recombination mechanism. However, the reason underlying a low efficiency of the classical gene targeting, implying the use of homologous recombination, may be a discrepancy between the goal of homologous recombination and its capabilities selected by the evolution to perform its own, although yet unknown function. The relevant published data allow for identification of certain factors that are critical for the work of homologous recombination mechanism.

The linear size of the DNA fragments delivered to the place where recombination complex is located is important for efficient operation of the homologous recombination mechanism. For example, a 100-fold exponential growth in gene targeting efficiency was recorded when increasing the length of homologous regions in the correction construct from 2 kbp to 14.5 kbp; however, the achieved efficiency-10⁻⁵ events of gene correction per cell remained yet insufficient for applying this method without selection [Deng, C. and Capecchi, M.K. Reexainination of gene targeting frequency as a function of the extent of homology between the targeted vector and the target locus. Mol. Cell Biol. 12, 3365-3371 (1992)]. The results on using small fragments homologous replacement (SFHR) for correction of a mutant gene presented a drastic contrast with the above data-the rate of correction amounted to 1-20% per cell [US, No. 5,470,577, 28.11.1995, Gruenert, D., Bruscia, E., Novelli, G., Colosimo, A., Dallapiccola, B., Sangiuolo, F., Goncz, K. Sequence-specific modification of genomic DNA by small DNA fragments. Clin. Invest. 112, 637-641 (2003)]. The gene targeting efficiency obtained in these experiments may mean that homologous recombination prefers to operate with either short fragments or form from short fragments the multimeric fragments, which are most efficient in the recombination events, via ligation of DNA fragments by ligase IV according to head-to-tail manner.

The necessary condition for obtaining of the recombinant product in mammalian cells is the length of the terminal homology, which amounts to about 200 bp [Lin, Y., Lukacsovich, T., Waldman, A.S. Multiple pathways for repair of DNA double-strand breaks in mammalian chromosomes. Mol. Cell Biol. 19, 8353-60 (1999), Nassif, N., Engels, W. DNA homology requirements for mitotic gap repair in Drosophila. Proc. Natl. Acad. Sci. USA. 90 (4):1262-6 (1993)].

The principal condition for performance of homologous recombination is a linear from of the delivered therapeutic DNA as a source double-stranded ends. Circular forms of therapeutic plasmids are virtually not involved into the homologous exchange. Generation of DSB and formation of double-stranded ends of DNA molecule locally activates and boosts the chromosomal and extrachromosomal recombination and gene targeting [Kucherlapati, R.S., Eves, E.M., Song, K.Y., Morse, B.S., Smithies, O. Homologous recombination between plasmids in mammalian cells can be enhanced by treatment of input DNA. Proc. Natl. Acad. Sci. USA. 81, 3153-3157 (1984)]. A recent publication in the *Nature* [Urnov, F.D., Miller, J.C., Lee, Y.L., Beausejour, C.M., Rock, J.M., Augustus, S., Jamieson, A.C., Proteus, M.H., Gregory, P.D., Holmes, M.C. Highly efficient endogenous human gene correction using designed zinc-finger nucleases. Nature. 435, 646-651 (2005)] reports using of this principle for induction of correction of IL2R□ gene, where a point mutation in exon 5 causes a mortal hereditary disease-severe combined immunodeficiency (SCID), linked to the X chromosome. The authors demonstrated a high, up to 20%, level of gene correction by transfection of the cells with genetic constructs expressing synthetic zinc-finger nucleases, capable of cleaving specifically the sequence in the vicinity of the mutation in IL2R□ gene in genomic DNA, and a plasmid carrying the non-mutant fragment of the same gene covering the region of SCID mutation.

Presumably, the ability of homologous recombination in principle to every efficiently perform gene correction is connected with the initiation of the processes by emergence of free double-stranded DNA ends in the nuclear space independently of the origin of these ends-whether they are the ends of a broken chromosome formed was a result of DSB or the double-stranded ends of DNA fragments delivered to the nucleus and representing typical ends-out structures. Any unmodified fragment of genomic DNA with its two ends homologous to two sequences of chromosomal DNA and the center part capable of substituting the corresponding genomic fragment and correcting a mutation is a typical ends-out construct. This means that the ends of the fragment will initiate the search for homology and homologous recombination with genomic DNA, which will lead to substitution of the genomic DNA fragment with the central part of the fragment delivered from the outward space to the cell. Under conditions of using of genetically correct exogenous DNA, these events will lead to correction of the genetic defect [Yakubov, L., Popova, N., Nikolin, V., Semenov, D., Bogachev, S., Oskina, I. Extracellular genomic DNA protects mice against radiation and chemical mutagens. Genome Biology. 5:P3 (2003)].

The blood plasma and interstitial fluids of mammals and humans always contain fragments of genomic DNA, formed as a result of genomic DNA fragmentation during natural cell death [Anke,r P., Zajac, V., Lyautey, J., Lederrey, C., Dunand, C., Lefort, F., Mulcahy, H., Heinemann, J., Stroun, M. Transcession of DNA from bacteria to human cells in culture: a possible role in oncogenesis. Ann. N Y Acad Sci. 1022, 195-201 (2004), Anker, P., Mulcahy, H., Chen, X.Q., Stroun, M. Detection of circulating tumour DNA in the blood (plasma/serum) of cancer patients. Cancer Metastasis Rev. 18, 65-73 (1999)]. These DNA fragments are involved in the constantly occurring DNA exchange via engulfment by actively dividing cells and delivery to cell compartments. The delivery of extracellular DNA is performed via an active receptor-mediated pinocytosis with involvement of specific membrane receptors [Yakubov, L.A., Deeva, E.A., Zarytova, V.F., Ivanova, E.M., Ryte, A.S., Yurchenko, L.Y., Vlassov, V.V. Mechanisms of oligonucleotide uptake by cells: Involvement of specific receptors? Proc. Nail. Acad. Sci. USA 86, 6454-6458 (1989), Doerfler, W., Remus, R., Muller, K., Heller, H., Hohlweg, U., Schubbert, R. The fate of foreign DNA in mammalian cells and organisms. Dev. Biol. (Basel), 106, 89-97, (2001)]. There are many methods for gene transfection; however, these methods provide only a compact packaging of therapeutic DNA necessary for their transportation by the considered receptor-mediated mechanism [Budker, V., Budker, T., Zhang, G., Subbotin, V., Loomis, A., Wolff, J.A. Hypothesis: naked plasmid DNA is taken up by cells in vivo by a receptor-mediated process. Gene Med. 2(2), 6-88, (2000), Satkauskas, S., Bureau, M.F., Mahfoudi, A., Mir, L.M. Slow accumulation of plasmid in muscle cells: supporting evidence for a mechanism of DNA uptake by receptor-mediated endocytosis. Mol. Ther. 4, 317-323 (2001)]. Creation of a high concentration of the fragmented genetically correct DNA in the blood plasma and interstitial fluids will result in appearance of the competition-based displacement of pathological DNA fragments circulating in the blood and appearing as a result of cancer cell death from the constant process of homologous exchange with the corresponding regions of chromosomes. A long-term duration of such effect will lead to restoration of the cell healthy genotype and reverse transformation to the norm [Yakubov, L., Popova, N., Nikolin, V., Semenov, D., Bogachev, S., Oskina, I. Extracellular genomic DNA protects mice against radiation and chemical mutagens. Genome Biology. 5:P3 (2003), Yakubov, L.A., Petrova, N.A., Popova, K.A., Semenov, D.V., Nikolin, V.P.,Oskina, I.N. Role of extracellular DNA in maintaining of stability and variability of cellular genomes. Dokl. Biokhim. Biofiz. 382, 31-34 (2002)].

The stated technical problem is solved basing on the developed theoretical concept of the turnover of extracellular DNA in the organism, which has found numerous expressional confirmations. The main theses of the concept of the turnover of extracellular DNA in eukaryotic organism are giver below.

Extracellular fragmented genomic DNA containing mutant sequences at the rate corresponding to the frequencies of the corresponding mutations in the cells is formed as a result of the programmed cell death, or apoptosis of cells, and is always present in the intercellular space and blood plasma [Anker, P., Mulcahy, H., Chen, X.Q., Stroun, M. Detection of circulating tumour DNA in the blood (plasma/serum) of cancer patients. Cancer Metastasis Rev. 18, 65-73 (1999), Giacona, M.B., Ruben, G.C., Iczkowski, K., Roots, T., Porter, D., Sorenson, G. Cell-free DNA in human blood plasma: length measurements in patients with pancreatic cancer and healthy controls. Pancreas. 17, 89-97 (1998)]. Cells are capable of engulfing the formed DNA or chromatin fragments via a receptor-mediated mechanism and transporting these fragments to cell nuclei [Yakubov, L.A., Deeva, E.A., Zarytova, V.F., Ivanova, E.M., Ryte, A.S., Yurchenko, L.Y., Vlassov, V.V. Mechanisms of oligonucleotide uptake by cells: Involvement of specific receptors? Proc. Nail. Acad. Sci. USA 86, 6454-6458 (1989), Shestova, O.E., Andreeva, A.Yu, Vlassov, V.V., Yakubov, L.A. Transportation of the complexes of oligonucleotides with cell surface proteins into the cell nucleus. Dokl. Ross. Akad. Nauk 368, 264-267, (1999)]. The delivered fragments recombine with the genomic DNA in the nuclei according to the mechanism of homologous recombination and are able to initiate this process themselves. Presumably, the combination of these three processes compose the mechanism capable of controlling the genetics of somatic cells at the level of overall organism by correcting mutations or inducing genetic changes in the cells using the extracellular DNA of tissue fluids and blood plasma as an external genomic standard. The work of such mechanism could form the background for the genome unity of the organism's cells. This mechanism could eliminate the emerged and fixed mutations as well as insert mutations into cells with the help of mutant sequences of extracellular DNA standard. If this mechanism could use the DNA standard virtually free of harmful mutations, its only function will be elimination of mutations from the cells of the body. Since the allelic genes usually highly homologous and frequently differ in a relatively small regions of changed sequences, the other aspect of the function of the putative mechanism could be substitution of sequences of the allelic genes in chromosomes with alternative sequences, similar to substitution of mutant sequences with non-mutant.

The stated technical problem is also solved by that the complete genome of an individual is introduced into the organism as a set of polynucleotide molecules that are fragments of genomic DNA, where it circulates, is engulfed by the cells, and reaches the nucleus, where it is deposited and involved in homologous recombination with cell genomic DNA. Thus, the possibility arises to correct genetic defects by introduction into the organism of a complete genome as a set of polynucleotide molecules that are fragments of genome DNA free of genetic defects as well as the possibility to provide an efficient treatment to the individuals suffering with diseases and disorders related to genetic mutations based on the method described in this invention. The treatment method proposed utilizes the fragments of genomic DNA comprising the complete genome of a physiologically and genetically healthy individual associated with the proteins of nuclear matrix. The size of exogenous fragments fits the size of the fragments circulating in the blood plasma of the treated organism which appeared due to apoptotic nucleolysis (1-30 nucleosome units). The prototypes use various artificial DNA constructs that does not represent a complete genome of an individual. The DNA is not associated with the proteins of nuclear matrix and does not fir the size of the natural apoptotic DNA of the organism, amounting to 1-30 nucleosome units.

The distinctive characteristic features of the proposed method for treatment of oncologic diseases induced as a consequence of multimutant events in the cell were studied using human breast adenocarcinoma cell culture (*in vitro*). The proposed method for treatment of oncologic diseases was studied in experiment with induced tumors in mice (*in vivo*). The maximally permissible amount of therapeutic DNA proposed for administration and specified in the declared treatment method was determined in tests for cytotoxicity performed using human cell culture (*in vitro*).

The delivery of fragmented genomic DNA of an exogenous origin into the inner cell compartments, determined in the proposed treatment method, was studied in a model of human breast adenocarcinoma cell culture.

### Example 1

Cell culture. Human breast adenocarcinoma (MCF-7) cells were cultivated in the RPMI 1640 medium supplemented with 10 mM L-glutamine and 50 µg/ml streptomycin (Sigma, USA) at 37°C in the presence of 5% fetal bovine serum (FBS; Biolot, Russia) in an atmosphere of 5% CO₂ to a density of 0.7 × 10⁷ cells per 4 wells of a 24-well plate.

Preparation of DNA and precursor. Human placental DNA (10 µg) fragmented to 500 bp was labeled by nick translation in the presence of Klenow fragment, three cold, and one hot dNTPs. The unincorporated precursors were removed by double precipitation with isopropanol according to the protocol described by Glover [Glover, D., 1988. DNA Cloning: A Practical Approach, IRL Press, Oxford, 1985. Translated under the title Klonirovanie DNK. Metody. Mir, Moscow]. The yield of labeled DNA upon purification amounted to 7-15 µg. DNA was dissolved in an appropriate volume of distilled water and added to wells of the plate (no more than 20 µl per well). Each well contained 250-350 µl of the medium. A 1-µl aliquot was taken from the working volume to determine radioactivity.

The aliquot of α-dNTP* was diluted in the appropriate volume of water, and 1 µl of the diluted triphosphate was sampled to count radioactivity. An approximately similar amount of α relative to DNA (with respect to radioactive count and volume) was added per each point.

The amounts of DNA (µg and cpm) and α-dNTP* (cpm) for the three experiments are listed in Table 1.

**Table 1 General characterization of the quantities of the material used in the experiments.**

| | Experiment II | | Experiment III | | Experiment IV | |
|---|---|---|---|---|---|---|
| | µg | cpm | µg | cpm | µg | cpm |
| DNA* | 1.4 | 1.08 × 10⁷ | 3.6 | 2.04 × 10⁷ | 2.5 | 1.8 × 10⁷ |
| α-dATP* amount (cpm) taken per point B cpm | | 0.9 × 10⁷ | | 2.0 × 10⁷ | | 1.4 × 10⁷ |

### RESULTS

Distribution of extracellular DNA with a length of 500 bp, represented by a pool of fragments comprising the complete human genome, over cell compartments

Figures necessary for quantitative assessment of experimental results:

The counts of human breast adenocarcinoma cells MCF-7 per experimental point were 0.7 × 10⁶.
According to the protocol for MCF-7, 10⁷ cells contain 60 µg DNA.
One cell contains 6 picogram of DNA.
The total DNA amount at experimental point was 4.2 g.
The amounts of labeled DNA added to experimental point are listed in Table 1. Radioactivity counts of the added DNA (cpm) are shown in Table 1.
Radioactivity counts of α-dNTP* per experimental point (cpm) amounted to the values listed in Table 1. The haploid human genome contains 3.3 × 10⁹ bp. The initial size of labeled DNA fragments added to the medium amounted to 500 bp.

Two stages of the culture growth (exponential phase and the phase of contact inhibition) and two types of electrophoretic assay (under native and denaturing conditions) were chosen for analyzing the behavior of extracellular DNA. Qualitative characteristics and quantitative parameters of extracellular DNA behavior in the cell were analyzed.

Qualitative behavior characteristics of labeled material (native conditions). Exponential phase of cell growth at the moment DNA and precursor are added to culture medium The fist page of figures contains electrophoretic pattern (right) and the corresponding X-ray pattern obtained by exposure to the same agarose blocks upon drying (left).

### Cytoplasmic fraction (upper group of blocks)

DNA enters the cell cytoplasm virtually immediately. At zero point, the low-molecular-weight fraction corresponding in its mobility to the initial labeled material is seen. The label is absent at the start of the zero point. All the rest samples display a considerable amount of labeled material at the start. This may suggest that the DNA forms high-molecular-weight complexes with components of the cytoplasm. An evident jellyfish of the precipitated DNA was undetectable. All the samples had DNA fractions with equal mobilities coinciding with the mobility of the initial labeled DNA added to the medium. This means that the freely migrating portion of the DNA delivered to the cytoplasmic fraction does not undergo any visible metabolic transformations. The other part of DNA that is found at the start was either transformed in a certain way or incorporated into high-molecular-weight complexes.

### Interchromosomal fraction (middle group of blocks )

DNA enters the interchromosomal fraction of the nucleus virtually immediately too. At the zero point, the low-molecular-weight fraction corresponding in its mobility to the initial labeled material is seen. Label is absent at the starts of the zero point and all the rest samples. The entire high-molecular-weight DNA fraction formed a lenticular sediment during centrifugation, which was undetectable in the nuclear sap. Change in DNA mobility is observed in all the samples except for the zero point. DNA forms a high-molecular-weight pool with a shape of "fuzzy cloud" rising from the lower part into the high-molecular-weight region of agarose block. In addition, several discrete size-dependent bands are seen on the background of the uniform cloud. The chromatin fraction, where an insignificant contamination with interchromosomal material is present (the chromatin sediment was not washed), displays an especially distinct banding pattern. This distribution pattern of the labeled material suggests the following events. Presumably, the fragmented DNA that entered the nucleus acts as a primer for synthesis of DNA strand. This manifests itself in emergence of a set of various-sized labeled DNA fragments, seen in the electrophoretic pattern as a fuzzy cloud of labeled material reaching a size of 10 kbp. Another possibility is that the DNA fragments are ligated at their ends. This assumption stems from the pattern of discrete labeled bands multiple to the initial DNA in their sizes. A combined variant of increase in the linear size of the extracellular DNA in the nuclear interchromosomal space is also possible.

Chromatin (lower group of blocks) . DNA (itself or the label in any other form) enters the nucleus virtually immediately and integrates into the fraction of nuclear chromatin. In the region of evident separation, a pronounced ladder of the fragments multiple to the initial DNA in their lengths is seen. (This material appears due to an insignificant contamination with the interchromosomal fraction, containing a high specific activity label.) α-dATP* (in all blocks, designated 180α)

α-dATP* is lacking in the cytoplasmic fraction. However, a labeled fragment with a size coinciding with the DNA used in experiment is present. In the interchromosomal fraction, it forms analogous, though less intensive fuzzy cloud, which completely corresponds morphologically to that seen in the analogous fraction with labeled DNA. The label is detectable in chromatin after 180 min. Other time points for α-dATP* were not monitored. The label counts in the samples of the culture medium at the point of 180 min are approximately equal for labeled DNA and α-dATP* (see Tables 1 and 2). However, the amount of labeled material detected at this experimental point with α-dATP* is approximately sixfold smaller compared with the variant with DNA.

Qualitative behavior characteristics of labeled material. All the quantitative estimations made based on comparison of the counts at the experimental points in various cell compartments are listed in Tables 1 2 and 3

**Table 2 The absolute counts (AC, cpm) and percent of the α-dATP*-labeled material added to culture medium.**

| α-dATP* 180 min | Experiment II Exponential phase | Experiment III Cell monolayer | Experiment IV Exponential phase |
|---|---|---|---|
| Cytoplasmic fraction (cpm) | 94 775 | 128 271 | 348 924 |
| % of the amount added to medium | 1.1 % | 0.64% | 2.49% |
| Interchromosomal fraction (cpm) | 31 328 | 13 886 | 128212 |
| % of the amount added to medium | 0.35% | 0.07% | 0.88% |
| Chromatin (cpm) | 5915 | 14689 | 32516 |
| % of the amount added to medium | 0.066% | 0.07% | 0.22% |

One point-180 min-is taken in the experiment

**Table 3 The quantitative characteristics of the labeled material (DNA*) content in various cell compartments depending on the time of its incubation with cells.**

| Incubation time | 0 min | | | 15 min | | | 30 min | | | 60 min | | | 120 min | | | 180 min | | |
|---|---|---|---|---|---|---|---|---|---|---|---|---|---|---|---|---|---|---|
| Experiment no. | II | III | IV | II | III | IV | II | III | IV | II | III | IV | II | III | IV | II | III | IV |
| Cytoplasmic fraction (absolute counts, cpm) | 74883 | 470 559 | 275 481 | 54230 4 | | | 67421 6 | | | 677 58 8 | 98042 1 | 85476 5 | | 960000 | 1 060 674 | 518282 | 863345 | 368101 |
| % of the amount added to medium | 0.7% | 2.3% | 1.5% | 5.0% | | | 6.2% | | | 6.3% | 4.8% | 4.7% | | 4.7% | 5.8% | 4.8% | 4.2% | 2.0% |
| Interchromoso mal fraction (absolute counts, cpm) | 11 460 | 3060 | 7330 | 83662 | | | 95821 | | | 97147 | 159 37 8 | 32200 8 | | 299 963 | 445 426 | 177 814 | 282 826 | 20 3421 |
| % of the amount added to medium | 0.1% | 0.015% | 0.04% | 0.77% | | | 0.89% | | | 0.9% | 0.78% | 1.75% | | 1.47% | 2.42% | 1.65% | 1.39% | 1.1% |
| Amount in µg according to % | 0.0014 | 0.0005 | 0.0025 | 0.01 | | | 0.013 | | | 0.0135 | 0.03 | 0.04 | | 0.053 | 0.061 | 0.023 | 0.05 | 0.028 |
| % of the genome | 0.066% | 0.016% | 0.083% | 0.33% | | | 0.43% | | | 0.45% | 1.0% | 1.33% | | 1.77% | 2.03% | 0.73% | 1.67% | 0.92% |
| Chromatin (absolute counts, cpm) | 8089 | 3691 | 2248 | 45 812 | | | 47684 | | | 49 394 | 296 771 | 12339 0 | | 242836 | 157124 | 37620 | 244937 | 118285 |
| % of the amount added to medium | 0.075% | 0.018% | 0.012% | 0.4% | | | 0.44% | | | 0.46% | 1.45% | 0.67% | | 1.19% | 0.85% | 0.35% | 1.2% | 0.64% |
| Amount in µg according to % | 0.0011 | 0.00065 | 0.0003 | 0.0056 | | | 0.006 | | | 0.0064 | 0.05 | 0.0168 | | 0.0428 | 0.021 | 0.0042 | 0.043 | 0.016 |
| % of the genome | 0.026% | 0.017% | 0.007% | 0.13% | | | 0.14% | | | 0.15% | 1.19% | 0.4% | | 0.95% | 0.5% | 0.1% | 1.02% | 0.4% |

To be noted that the material in all the procedures is present an absolute quantities; therefore, it is appropriate to compared the data and relate the quantities to the label initially introduced into the culture medium.
The experiments gave an unanticipated result that, first, α-dATP* was conveyed to all the cell compartments not as a monomer, but within DNA fragments of about 500 bp long. Further, this DNA is involved in all the processes similar to the DNA added to the medium. In all respects, the activity of α-dATP* utilization is severalfold less intensive compared with the utilization of labeled extracellular DNA. Then it was found that extracellular DNA was virtually immediately (less than during 1 min if the cells were actively dividing) delivered to all the cell compartments analyzed (cytoplasm and nuclear space) and integrated into the chromatin. In the cytoplasm of actively dividing cells, approximately equal amount of labeled DNA was present at all the experimental points except for zero point. The label accumulated in the nuclear compartments depending on the time of exposure to labeled DNA present in the culture liquid. DNA was present in the interchromosomal space of the nucleus as fragments of various lengths. The size of the fragments at the zero point in the actively dividing cells corresponds to the size of extracellular DNA added to the medium. In all the rest variants, the linear DNA size increased to approximately 10 kbp. Moreover, the "ladder" of the bands detected suggests that a part of the fragments were ligated in a head-to-tail manner. In cells of the culture at the stage of contact inhibition, DNA processing (denaturing conditions data not shown) displayed a distinct dynamics. Presumably, the metabolic processes at this stage of cell culture growth are slowed down, and we succeeded in detecting the dynamic stage of the process of forming multimers of the initial DNA fragments in the nucleus. Increase in the linear size of the DNA molecules delivered to the nuclear space may result from the specific DNA ligase IV activity, involved in repair events (for references see [Lees-Miller, S.P., Meek, K. Repair of DNA double strand breaks by non-homologous end joining. Biochimie 85, 1161-1173 (2003)]).

The data reported imply that in the proposed method for treatment of oncologic diseases, a certain amount of DNA is delivered to the nucleus of the cancer cell, is constantly present in the interchromosomal space, and is integrated into the genome. In the interchromosomal space, DNA undergoes the changes that increase its linear size to bout 10 kbp.

The feasibility of correcting mutations by the effect of extracellular DNA on cancer cells, considered in this patent application and in the proposed method for treating cancer diseases, was studies using a mutant caspase 3 gene from human adenocarcinoma. The studies performed demonstrated both the activity 15 tion of caspase 3 gene in the cells of human breast adenocarcinoma MCF-7 and apoptotic development pathway of the previously cancer cells under the effect of extracellular DNA.

### Example 2

Cell culture and cultivation conditions

Human breast adenocarcinoma (MCF-7) cells and mouse sarcoma (L929) cells were obtained from the Institute of Cell Cultures, SRC VB Vector. The cells were cultivated in the RPMI 1640 medium supplemented with 10 mM L-glutamine and 50 µg/ml streptomycin (Sigma, USA) at 37°C in the presence of 5% fetal bovine serum (FBS; Biolot, St. Petersburg, Russia) in an atmosphere of 5% CO₂. For transfection MCF-7 cells were cultivated in the RPMI 1640 medium supplemented with 10 mM L-glutamine and 50 µg/ml streptomycin in the presence of 5% fetal bovine serum (FBS) and 0.1-0.2 mg/ml of human placenta DNA. The time of transfection varied from 5 to 40 days as in indicated in figure captions.

PCR analysis. PCR analysis was performed according to standard protocols using Medigen (Russia) and BioRad (USA) kits. Real-time PCR assay was performed according to the protocol of BioRad (USA).

The following primers were synthesized for analysis of mRNA of caspase 3 gene:
- Pr. 1: GAATGACATCTCGGTCTGGTA (21);
- Pr. 2: TGAATAATGAAAAGTTTGGGT (21);
- Pr. 3: CTCTGGAATATCCCTGGACAACA (23); and
- Pr. 4: CATCACGCATCAATTCCACAAT (22).

The amplified DNA fragments were separated in 2% agarose gel and stained with ethidium bromide.

### RNA isolation from cell culture

A Total RNA Purification Kit (V-gene Biotechnology Limited) was used for RNA isolation. The procedure was performed according to the below protocol.

PCR analysis of mRNA of the MCF-7 cells after a long-term exposure to human extracellular DNA

Detection in the extract of the cells treated with human DNA either the enzyme caspase 3 or the corresponding mRNA with the rescued mutation in exon 4 could represent a direct confirmation of the homologous integration of the therapeutic fragment into the chromosome of mutant cells. We chose the approach connected with detection and analysis of the corresponding mRNA that would allow for a detection of the sites of exon splicing and the presence of rescued mutation in mRNA by direct sequencing. Using RT (reverse transcription)-PCR procedure, we amplified the mRNA region containing exons 4, 5, and 6. Here the oligonucleotide homologous to the 47-bp region from exon 4 (primer 3), which is absent in the genome of MCF-7 cells, was used as a forward primer. The oligonucleotide complementary to the end part of exon 6, whose last nucleotide corresponded to the last nucleotide of exon 6 was used as a reverse primer. Initially, total RNA was isolated from the MCF-7 cells incubated for 15 days with human DNA, and cDNA was synthesized. Primes 2 and 3 (Pr. 2 and Pr. 3) were used to detect the mRNA region corresponding to caspase 3 gene and containing repaired deletion in exon 4. The amplification produced the fragment sought for with a size of 431 bp, including the spliced exons 4 (lacking 20 first nucleotides), 5, and 6 (Fig.2). Detection of this fragment in the fraction of total RNA means that the total RNA contains the mRNA of caspase 3 gene with a repaired 47-bp deletion. To control the correctness of amplification of the 431-bp fragment, two expected PCR products (the fragments with a length of 236 and 304 bp) were obtained using two inner primers (Pr 1 and Pr. 4), two terminal primers (Pr. 3 and Pr. 4), and 431-bp DNA fragment as a template (Fig.3). Sequencing of both fragments allowed us to make the following inferences. The 431-bp fragment is a cDNA copy of the region of caspase 3 mRNA containing spliced exons 4 (lacking 20 first nucleotide), 5, and 6 (Fig. 3). The left part of 431-bp fragment is the repaired region of exon 4 containing the lacking 47 nucleotide (the region 320-367 of caspase 3 mRNA), which are absent in the initial MCF-7 culture, correctly spliced with the next two exons. This demonstrates that the therapeutic fragment was homologously integrated into the defect region of exon 4. The integration followed the mechanism described in [Langston, L.D., Symingtom, L.S. Gene targeting in yeast is initiated by two independent strand invasions. Proc NM Acad Sci USA. 101(43): 15392-15397 (2004), Li, J., Read, L.R., Baker, M.D. The mechanism of mammalian gene replacement is consistent with the formation of long regions of heteroduplex DNA associated with two crossing-over events. Mol. Cell Biol. 21(2):501-510 (2001)], when the middle part of the integrated fragment is not important for the process of integration. When comparing the splices sequence of 431-bp fragment, containing exons 4, 5, and 6 with the original sequence, we detected two insertions each of 6 nucleotides (the region 370 and 390 of caspase 3 mRNA). Both insertions do not cause a frameshift. In the experimental sequence, the cysteine absent at position 370 of caspase 3 gene is, presumably compensated for by the cysteine at position 350. Appearance of these two insertions is connected with either variation in exon 4 of caspase 3 gene or is a consequence of integration of the extrachromosomal fragment into the chromosome. The rest part of the sequence contains few insignificant substitutions. A virtually complete homology of the rest part of the sequenced template, where the exon splicing sites correspond to the sequence in GenBank, confirm the correctness of splicing. Detection of the repaired mRNA and demonstration of an active apoptosis with a characteristic nucleosome-sized fragmentation indicated the appearance of a functionally active enzyme.

It is known that caspase gene is localized to chromosome 4, has a size of 21 751 bp, and is composed of eight exons. Two expression products of this gene may exist during the cell metabolism: the transcripts of 17 and 19 kbp and their protein products [Wolf, B.B., Schuler, M., Echiverri, F., Green, D.R.. Caspase-3 is the primary activator of apoptotic DNA fragmentation via DNA fragmentation factor-45/inhibitor of caspase-activated DNase inactivation. J. Biol. Chem. 274, 30651-30656 (1999)]. A complex process of mRNA translation, when eight exons are spliced, is required for appearance of the functional enzyme caspase 3. The 47-bp deletion in exon 4 leads to omittance of exon 4 during splicing an impairment of RNA synthesis and expression of the active enzyme. The DNA present in culture medium is fragmented hydrodynamically to a size of 500-1000 this means that the fragments able to be used for synthesis of the full-sized product giving the functional enzyme are absent in principle. The probability of ligation of the necessary fragments in the correct orientation that led to restoration of the open reading frame for caspase 3 gene and, thereby, the formation of the extrachromosomal template appropriate for synthesis of the enzyme is vanishingly small. To our mind and according to the results of the experiments performed, the integration into the genome of the healthy fragment that reversed the caspase 3 gene to the norm is the most probable ending of the events related to transportation of extracellular DNA in these experiments.

Thus, the results of the studied performed demonstrate that the new trait appears in the cell culture, the exclusive carrier of which was the extracellular fragmented allogeneic DNA.

The data reported imply that in the proposed method for treating oncologic diseases, the fragments of extracellular DNA delivered by natural cellular mechanism find their homologous segments on the chromosomes and recombine with them. Moreover, if a chromosome region carries a mutation, the homologous extracellular DNA fragment is capable of correcting this mutation due to homologous substitution of the mutated region.

The proposed method for treating oncologic diseases was studied in an *in vivo* system using experimental tumors of mice.

### Example 3

The experiments were performed using A/Sn, CBA/Lac, C57BL/6, and ICR mice with an age of 3-4 months obtained from the animals breeding facility of the Institute of Cytology and Genetics, Siberian Branch of the Russian Academy of Sciences. Three strains of continuous tumors were used in the work: Ehrlich tumor, GA-1 hepatoma, and Lewis tumor. Ehrlich ascites and Lewis lung carcinoma wee obtained from the Institute of Oncology, Tomsk Scientific Center, Siberian Branch of the Russian Academy of Medical Sciences. GA-1 hepatoma was primarily induced in A/He mice with *ortho*-aminoazotoluene and is maintained in ascites form in A/Sn mice. The tumor is inducible by a subcutaneous or intramuscular inoculation and grows at the inoculation site as a solid mode with numerous metastases into the liver.

Placentas of healthy women in childbirth were the source of human DNA; a mixture of organs (liver, kidneys, spleen and thymus) of mouse DNA. DNA was isolated using phenol-free method (laboratory regulations of the Institute of Cytology and Genetics). This method allows for production of a full-fledged genomic DNA with preservation of the fragments that under *in vivo* conditions are stably associated with nuclear matrix proteins. DNA was fragmented in an ultrasonic disintegrator to produce a mixture of fragment with a size of 200-6000 bp. The preparations were stored packed as aliquots in a refrigerator at a temperature of 18°C. Before using, the necessary quantity of DNA was thawed and adjusted its concentration with physiological saline solution.

The data were processed statistically with the computer program Statistika. The significance of the distinctions was estimated according to Student's test and Whitny-Wilcoxon test.

The experiments performed demonstrate that the preparations of fragmented genomic DNA display the ability to slow down the growth of these tumors and have a moderate inhibiting effect on development of metastases (Table 4 and 5; Fig. 7).

**Table 4 The data on the lifespan of the mice carrying GA-1 tumor in the control group and the groups that received DNA.**

| Effect | Experiment 1 | | | Experiment 2* | | |
|---|---|---|---|---|---|---|
| | Number of mice | Average lifespan, days | p | Number of mice | Average lifespan, days | p |
| Control | 20 | 30.9 ± 1.24 | - | 10 | 45.3 ± 1.72 | - |
| Human DNA s.c. | 19 | 30.5 ± 0.77 | - | 10 | 47.4 ± 1.65 | - |
| CBA DNA i.p. | 20 | 31.8 ± 1.53 | - | 10 | 48.1 ± 1.67 | - |
| CBA DNA s.c. | | | | 10 | 52.9 ± 2.52 | <0.05 |
| A/Sn DNA s.c. | 20 | 35.5 ± 1.73 | <0.05 | | | |

| | | | | | | |
|---|---|---|---|---|---|---|
| *The inoculation dose in the first experiment amounted to 1 million cancer cells; in the second, to 0.5 million. | | | | | | |

**Table 5 The data on the effect of fragmented DNA on the growth and metastases formation of Lewis carcinoma.**

| Group | *n* | Average tumor weight, g | p | Lung metastases | | |
|---|---|---|---|---|---|---|
| | | | | Number of mice with metastases | Average number of metastases | Index of metastasis inhibition |
| 1. Control | 9 | 7.4 ± 0.40 | - | 8 (88.9%) | 28.2 ± 4.95 | - |
| 2. Fragmented DNA, 100 µg | 10 | 7.2 ± 0.26 | - | 10 (100%) | 23.1 ± 5.06 | 18.3 |
| 3. Fragmented DNA, 200 µg | 10 | 6.2 ± 0.18 | < 0.01 | 7 (70%) | 19.0 ± 4.97* | 32.9 |

| | | | | | | |
|---|---|---|---|---|---|---|
| *The data were statistically processed according to Whitny-Wilcoxon test; the differences with the control are statistically significant (p < 0.001). | | | | | | |

The cytotoxic and aberrant effects of DNA at a dose determined in the treatment method proposed were assessed in the model of human hTERT-BJ1 cell culture.

### MATERIALS AND METHODS

Solution of the fragmented DNA at a concentration of 1.695 mg/ml was used in the experiments. The DNA isolated from salmon roe under the same conditions was used as a reference preparation. The culture of diploid hTERT-BJ1 cell line (CLONTECH Laboratories, Inc. Catalog# C 4001-1) was used. The cells at an equal concentration were plated into six-well plates. After 24 h, the wells were supplemented with human or salmon DNA at a concentration of 300 or 30 µg/ml. the cell counts were determined in a Goryaev chamber 48 and 72 h after addition of DNA. Statistical processing of the data was performed using the Origin program.

To assess the chromosome rearrangements, the cells exposed to human or salmon DNA were fixed on slides and stained conventionally according to Giemsa.

### RESULTS

The data on cytotoxic effect of the fragmented DNA isolated from different sources and applied at different doses. Analysis of the data obtained demonstrated that introduction of both human and salmon DNA at a concentration of 300 µg/ml inhibited the growth of the couture on day 3 after adding. The concentration of DNA preparations of 30 µg/ml had no statistically significant effect on the growth of hTERT-BJ1 cell culture

**Table 6 The data on the effect of different concentrations of extracellular DNA on division rate of hTERT-BJ1 human cell culture.**

| **300** µ**g/ml** | **Control** | **Human DNA** | **Salmon DNA** |
|---|---|---|---|
| 2 days n = 4 | 100 +\- 5.82 | 89.78 +\- 15.84 | 83.11 +\- 23.16 |
| 3 days *n* = 3 | 100 +\- 17.55 | 66.46 +\- 18.99 | 51.27 +\- 16.55 |
| The difference is statistically significant: | | 100 +\- 17.55 and 66.46 +\- 18.99 | |
| | | 100 +\- 17.55 and 51.27 +\- 16.55 | |
| | | 83.11 +\- 23.16 and 51.27 +\- 16.55 | |
| 2 days n = 5 | 100 +\- 5.82 | 96.856 +\- 7.64 | 103.29 +\- 2.97 |
| 3 days n = 2 | 100 +\- 21.38 | 68.62 +\- 4.02 | 75.4 +\- 9.2 |

| | | | |
|---|---|---|---|
| No statistically significant differences X ± se p < 0.05 t-test | | | |

The data on aberrant effect of the fragmented DNA isolated from different sources

Analysis of the data obtained demonstrates that an increased level of chromosome aberrations was observed at a dose of 300 µg/ml of human or salon DNA. However, the xenogeneic DNA had a higher aberrant effect compared with the allogeneic human DNA

**Table 7. The analyzes rate of structural chromosome rearrangements induced in hTERT-BJ1 human diploid cell culture treated with extracellular DNA at a dose of 300 µg/ml for 2 days.**

| | Number of metaphases | | | | | | |
|---|---|---|---|---|---|---|---|
| | Analyzed | Without structural aberrations | With structural aberrations | With chromosome breaks | With fragments | With double breaks | With dicentrics |
| Treated human DNA | 119 | 106 (89%) | 13 (11%) | 3 | 7 | 2 | 1 |
| Treated salmon DNA | 107 | 87 (81%) | 20(19%) | | 15 | 3 | 2 |
| Intact | 123 | 118 (95%) | 5 (4%) | | 5 | | |

It was shown experimentally that the fragmented DNA at a concentration equal to 300 µg/ml or higher may inhibit cell growth and induce chromosome rearrangements. The concentration of 30 µg/ml had no cytotoxic effect. The recommended dose of DNA preparations used for therapeutic purposes is 5 µg/ml for single administration. This corresponds to 1 mg of DNA per 1 L of blood, or working concentration of 1 µg/ml, which is 300-fold lower that the concentration that caused undesirable effects and 30-fold lower that the concentration of exogenous DNA displaying no cytotoxic effect on the hTERT-BJ1 culture of human cells.

Thus, the proposed use of fragmented DNA for the treatment of oncologic diseases provides the required result, as it expands the area of method application and treatment of patients with oncologic diseases; moreover, this scheme allows the cause of the disease to be treated, that is, the mutations in the oncogenes and oncosuppressor genes to be corrected and heterozygous alleles to be introduced into the homozygotized genes of the cell.

## Claims

1. Use of fragmented DNA in manufacture of a medicament for the treatment of oncologic diseases, wherein the fragmented DNA is fragments of homologous DNA with a biologically active size and comprising the complete genome of a physiologically and genetically healthy donor.

2. Use of claim 1, wherein the fragmented DNA is used in an amount which is equal to or exceeds the amount of the own DNA in the blood plasma and tissue fluids, but does not exceed the maximal permissible dose, amounting to 30 µg/ml.
